(19) Europäisches Patentamt
European Patent Office
Office européen des brevets

(11) EP 3 231 454 B1

(12) EUROPEAN PATENT SPECIFICATION

(45) Date of publication and mention of the grant of the patent:
**22.09.2021 Bulletin 2021/38**

(51) Int Cl.:
*A61L 27/16* (2006.01) *A61L 27/54* (2006.01)
*A61L 24/00* (2006.01) *A61L 24/06* (2006.01)

(21) Application number: **16382170.5**

(22) Date of filing: **14.04.2016**

(54) **BONE CEMENT COMPRISING MICROENCAPSULATED ANTIMICROBIAL**

KNOCHENZEMENT MIT MIKROVERKAPSELTEM ANTIMIKROBIOTIKUM

CIMENT OSSEUX COMPRENANT UN ANTIMICROBIEN MICROENCAPSULÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**18.10.2017 Bulletin 2017/42**

(73) Proprietors:
- **UNIVERSIDAD PONTIFICIA COMILLAS 28015 Madrid (ES)**
- **Inescop Instituto Tecnologico de calzado y conexas 03600 Elda Alicante (ES)**
- **Carbo Laso, Esther 28006 Madrid (ES)**
- **Sanz Ruiz, Pablo 28002 Madrid (ES)**
- **Vaquero Martin, Javier 28034 Madrid (ES)**

(72) Inventors:
- **CARBO LASO, Esther 28006 Madrid (ES)**
- **SANZ RUIZ, Pablo 28002 Madrid (ES)**
- **VAQUERO MARTÍN, Javier 28034 Madrid (ES)**
- **DEL REAL ROMERO, Juan Carlos 28015 Madrid (ES)**
- **BALLESTEROS IGLESIAS, María Yolanda 28015 Madrid (ES)**
- **PAZ JIMÉNEZ, Eva 28015 Madrid (ES)**
- **ARÁN AIS, Francisca 03600 Elda, Alicante (ES)**
- **PÉREZ LIMIÑANA, María Ángeles 03600 Elda, Alicante (ES)**
- **SÁNCHEZ NAVARRO, María Magdalena 03600 Elda, Alicante (ES)**

(74) Representative: **Pons**
**Glorieta Rubén Darío 4**
**28010 Madrid (ES)**

(56) References cited:
**WO-A1-99/36057 WO-A1-2011/088296**
**US-A1- 2013 011 453 US-A1- 2015 093 443**

- **LI H ET AL: "Preparation, characterization and in vitro release of gentamicin from PHBV/wollastonite composite microspheres", JOURNAL OF CONTROLLED RELEASE, ELSEVIER, AMSTERDAM, NL, vol. 107, no. 3, 20 October 2005 (2005-10-20), pages 463-473, XP027664115, ISSN: 0168-3659 [retrieved on 2005-10-20]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 231 454 B1

## Description

The present invention relates to bone cements with microencapsulated antimicrobials. Therefore, the invention may be categorised under the field of medicine, specifically drugs for preventing and treating periprosthetic infections.

## STATE OF THE ART

Since the introduction of polymethylmethacrylate (PMMA) as bone cement in the 60s, it has proven to be an indispensable element in orthopaedic surgery and particularly in prosthetic replacement surgery.

For most applications, bone cements have an acrylic base. Typically, bone cement comprises two components: a dry powder component and a liquid component, which are mixed together in the operating room to obtain the cured cement. Therefore, setting time is of great importance in these materials. The powder component normally contains an acrylic polymer, such as for example polymethylmethacrylate. In addition, it usually comprises a polymerisation initiator such as benzoyl peroxide. It may also include a radiopaque agent such as barium sulphate or zircon oxide or other types of solid additives with different functions. The liquid component normally contains the monomer which is mixed with the acrylic polymer powder. The monomer may be, for example, methyl methacrylate (MMA). The liquid component also contains a polymerisation reaction accelerator, such as (e.g.: N,N-dimethyl-p-toluidine). A stabiliser, such as hydroquinone, can be added to the liquid component to prevent the premature polymerisation of the liquid monomer. Once the two components have been mixed together, setting is completed between 5 and 20 minutes.

Bone cements are used to fix prostheses. They are also used as spacers when a prosthesis is removed due to a periprosthetic infection. In both cases, but particularly in the second, bone cements are usually loaded with antibiotics or other drugs for treating and preventing infections.

A periprosthetic infection is a serious complication whose prevalence has increased in the last decades due to the growing number of arthroplasties performed. Although its incidence is low, its prevalence is increasing basically due to the longer duration of the implants and to the greater life expectancy of the patients. Most of the infection-causing microorganisms are biofilm producers, which add another difficulty to microbiological diagnosis and treatment, complicating the action of the antibiotics. The biofilm is responsible for obstructing penetration by the antibiotics, since it acts as a barrier, protecting the bacteria of the immune system, whereupon the bacteria in its interior adopt a planktonic phase. The active doses of in-vitro antibiotic compared to a bacterium with a mature biofilm are 200 to 1,000 times greater than normal. This renders the treatment of prosthetic infections with systemic antibiotics impossible, because the necessary doses for achieving an effective concentration around the implant compared to microorganisms in biofilm would be toxic for the patient.

Two-stage reloading is the gold standard in tardy chronic infection, implantating, after removing the prosthesis, an antibiotic-loaded bone cement spacer, which makes it possible, in addition to maintaining the joint space, to reach high local antibiotic concentrations. Some bone cements are marketed together with antibiotics while in other cases the antibiotic is added to the powder component before final mixing. Once the cement has set, the antibiotics elute from the surface and through the pores and micro-cracks of the cement. The elution of the antibiotic of the bone cement is characterised in that it has a very high initial peak and must exceed the minimum inhibitory concentration (MIC) and the minimum bactericidal concentration (MBC). The antibiotic elution continues for a certain time although to a lesser degree, said curve being typical for all bone cements.

The bone cement works as a vehicle to release high doses of intra-articular antibiotic, without the toxicity and adverse effects that would imply the achievement of said concentrations via systemic administration. However, at times the use of certain antibiotics in cements is not recommended due to the reduction in their mechanical properties and to the appearance of resistance to antibiotics. Despite this, the use of the antibiotic-loaded cement in the primary prophylaxis of the prosthetic infection is common practice in the United Kingdom, Scandinavia and Western Europe that is being transmitted to the United States. Parvizi et al. concluded that the use of antibiotic-loaded cement as prophylaxis lowers the rate of infection in primary arthroplasties by 50% and in revision after aseptic loosening by 40% (Acta orthopaedica, 79(3), 335-41, 2008). US2015/0093443 A1 discloses a bone cement comprising microparticles loaded with an antibiotic such as rifampin. The bone cement can be a PMMA bone cement which is formed by mixing the powder component with the drug loaded microparticle and adding a monomer comprising a liquid methacrylate monomer.

Rifampicin has shown excellent activity against staphylococci in biofilm and is commonly used via systemic administration in periprosthetic infections; however, to date the production of bone cement (PMMA) that polymerises within a reasonable timeframe and having appropriate mechanical properties for use in clinical practice has not yet been achieved. It has been demonstrated that rifampicin inhibits or delays MMA, which lengthens bone cement setting time and also affects its mechanical properties.

Another problem with drug-loaded cements is the difficult elution of the antibiotics, which is closely related to the water absorption properties of the cement in relation to time and distance from the cement surface. The antibiotic diffusion rate depends on various factors, such as the chemical composition of the cement, the cement/bone interface

surface and the handling of the cement.

Therefore, alternative bone cements having good mechanical properties and acceptable setting times are required for use in the operating room and good elution properties.

## DESCRIPTION OF THE INVENTION

The present invention relates to bone cements comprising microcapsules containing rifampicin, rifabutin, rifapentine, rifalazil, and any combination thereof, wherein the bone cement is an acrylic-based bone cement with a polymethylmethacrylate base and wherein the microcapsules are formed by alginate and are obtained by ionic gelification.

The present invention has the following advantages:

- The microencapsulation of rifampicin and subsequent incorporation to the bone cement enables controlled release thereof in terms of time and place.
- The microencapsulation of rifampicin in alginate or PHBV (polyhydroxybutyrate-polyhydroxyvalerate), not part of the claimed invention, allows the bone cement to set completely within surgically reasonable timeframes and comparable to those of the control cement (without antibiotic).
- The bone cement that comprises the microcapsules has good mechanical properties after 45 minutes of setting, compared to the cement that contains unencapsulated rifampicin.
- The alginate and PHBV microcapsules with rifampicin show good elution in PBS (phosphate buffer with similar characteristics to the physiological medium). The PHBV microcapsules are not part of the present invention.
- The biocompatible and biodegradable microcapsules.
- The microencapsulation of rifampicin in alginate shows good encapsulation performance, with less PHBV content.
- Cements containing alginate and PHBV microcapsules have shown good results in microbiological assays with *S. aureus* and superior results with alginate.

Therefore, a first aspect of the present invention relates to bone cement comprising microcapsules containing an antimicrobial selected from rifampicin, rifabutin, rifapentine, rifalazil, and any combination thereof.

The microcapsules are formed by alginate.

The aforementioned antimicrobial agents cannot be added directly to the bone cement, since it prevents it from setting, or interact with the bone cement such that elution does not occur. Surprisingly, encapsulating these antimicrobials in microcapsules enables elution thereof without significantly affecting the mechanical properties of the cement.

Bone cements transfer the loads from the implant to the bone, filling in the spaces therebetween and absorbing impacts, damping the transfer of forces and distributing them throughout the interface. Bone cement is understood to be polymer materials which are used for the purpose of fixing prostheses or as spacers when a prosthesis is removed due to infection.

Microencapsulation is a widely known technique which is defined as the process of coating substances in the form of particles or liquid globules with materials of different nature, generally polymers, to obtain micrometric-sized particles. Therefore, the microcapsules are micrometric-sized particles with a fatty acid or polymer coating which contain an antimicrobial.

Antimicrobial is understood to be the antimicrobial compound and also its pharmaceutically acceptable salts.

Rifampicin, rifabutin, rifapentine and rifalazil are antibiotics belonging to the rifamicin family. The following antimicrobials are not comprised in the presently claimed bone cement.

Imidazole antifungicides are compounds derived from imidazole and comprise bifonazol, butoconazol, clotrimazol, econazol, fenticonazol, isoconazol, ketoconazol, luliconazol, miconazol, omoconazol, oxiconazol, sertaconazol, sulconazol, tioconazol and any combination thereof.

Triazole antifungicides are compounds derived from triazole and comprise albaconazol, efinaconazol, epoxiconazol, fluconazol, isavuconazol, iltraconazol, posaconazol, propiconazol, ravuconazol, terconazol, voriconazol and any combination thereof.

Carbapenem antibiotics are antibiotics with the general formula (I):

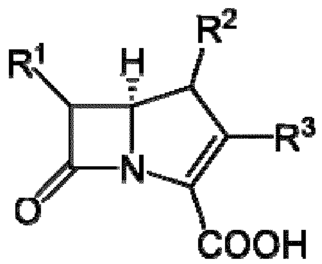

(I)

Carbapenem antibiotics are selected from imipenem, meropenem, ertapenem, faropenem, doripenem, panipenem and any combination thereof.

The bone cement as described earlier is characterised in that the microcapsules are formed by alginate.

Alginate is an anionic polysaccharide consisting of an unbranched binary copolymer of β-D-mannuronic (M) acid and α-L-guluronic (G) acid, structured in sequences of MM and MG blocks joined by β(1-4) linkages, and GG blocks joined by α(1-4) linkages. The M block regions correspond to linear chains, while the G blocks have a loop-shaped structure. Alginate is understood to be alkaline salts and alkaline earth metals of alginic acid, preferably alkaline earth metals. The alginate preferably comprises calcium alginate.

Microcapsules of alginate and other polyelectrolytes such as chitosan, carboxymethylcellulose (CMC), carrageenans, pectin, gellan gum, cellulose sulphate, polyphosphazenes, guar gum, xanthan gum and gum Arabic can be synthesised by ionic (ionotropic) gelification. This method is based on the cross-linking capacity of polyelectrolytes to form hydrogels in the presence of counter-ions. For example, in the case of alginate, the procedure consists of dissolving the antimicrobial to be encapsulated in an aqueous solution of sodium alginate. Subsequently, said mixture is added to the aqueous solution containing the counter-ion (such as a $CaCl_2$/chitosan solution). The addition can be performed by means of different procedures such as syringe drip, various vibration systems, air spraying to achieve extrusion of the polysaccharide solution which make it possible to obtain smaller particle sizes. When the drop of sodium alginate comes into contact with the solution containing the counter-ions (generally $Ca^{2+}$), it produces the instant gelification of the polysaccharide and gives rise to the formation of a membrane or coating. The ionic gelification method can be carried out directly by dripping the polysaccharide solution onto the counter-ion or indirectly, also called internally, when the counter-ion is released by an insoluble complex present in the polysaccharide solution.

In order to achieve reticulation of the polyelectrolyte and control factors such as membrane porosity and obtain different profiles and active substance release kinetics, counter-ions such as $Ca^{2+}$, tripolyphosphate (TPP), $Al^{3+}$, $K^{+}$, amines, poly-L-lysine, chitosan, polymethylene-co-guanidine and gelatin are used.

The cement comprising alginate microcapsules has shown good results. The encapsulation yields are high, with high elution values and the mechanical properties of the bone cement are not undermined.

The antimicrobial is selected from rifampicin, rifabutin, rifapentine rifalazil, and any combination thereof; more preferably, the antimicrobial is rifampicin.

The bone cement as described earlier is characterised in that it is acrylic-based bone with a polymethylmethacrylate base. Acrylic-based bone cement is understood to be a self-polymerisable biomaterial at room temperature composed of a powder fraction consisting of an acrylic polymer, preferably methyl methacrylate together with a polymerisation initiator, such as benzoyl peroxid, and a liquid fraction formed by a methacrylate copolymer in the form of a monomer (e.g.: methyl methacrylate, butyl methacrylate, etc.) stabilised by a polymerisation inhibitor such as hydroquinone and together with a polymerisation reaction activator such as an aromatic amine (N,N-dimethyl-p-toluidine). When the monomer and polymer come into contact, the N,N-dimethyl-p-toluidine activates the benzoyl peroxide and the resulting benzoyl free radicals initiate the polymerisation of the monomer.

Normally, bone cements are usually prepared in the operating room. They are prepared by mixing the component in powder form, which is usually mainly polymethylmethacrylate (PMMA), some formulations of which contain other types of acrylic polymers or copolymers, and the liquid component that comprises the methyl methacrylate monomer.

In another embodiment of the first aspect of the present invention, the microcapsules have a diameter between 2 μm and 2 mm, preferably between 1 μm and 1 mm, more preferably between 10 μm and 500 μm.

In another embodiment of the first aspect of the present invention, the concentration of the microcapsules is between 0.1% and 20% by weight with respect to the total weight of the bone cement, preferably between 0.1% and 10% by weight. When the bone cement is used as a spacer, it may be of interest to use a high concentration of microcapsules, despite the loss of mechanical properties.

In another embodiment of the first aspect of the present invention, the concentration of the antimicrobial is between 0.01 and 10% by weight with respect to the total weight of the bone cement, preferably between 0.05% and 5% by weight.

The microcapsules are formed by alginate and the concentration of the antimicrobial is between 0.01 and 10%

by weight with respect to the total weight of the bone cement, preferably between 0.05% and 5% by weight.

A second aspect of the present invention relates to bone cement, as described earlier, for use in therapy.

A third aspect of the present invention relates to bone cement, as described earlier, for use in the treatment and/or prevention of prosthetic infections.

In an embodiment of the third aspect of the present invention, the prosthetic infection is caused by microorganisms of genera selected from *Staphylococcus, Propionibacterium, Enterococcus, Streptococcus, Alishewanella,Alterococcus, Aquamonas, Aranicola, Arsenophonus, Azotivirga, Blochmannia, Brenneria, Buchnera, Budvicia, Buttiauxela, Cedecea, Citrobacter, Dickeya, Edwardsiella, Enterobacter, Erwinia, Escherichia, Ewingella, Grimontella, Hafnia, Klebsiella, Kluyvera, Leclercia, Leminorella, Moellerella, Morganella, Obesumbacterium, Pantoea, Paracolobactrum, Pectobacterium, Phlomobacter, Photorhabdus, Plesiomonas, Pragia, Proteus, Providencia, Rahnella, Raoultella, Salmonella, Samsonia, Serratia, Shigella, Sodalis, Tatumella, Trabulsiella, Wigglesworthia, Xenorhabdus, Yersinia, Yokenella, Corynebacterium, Pseudomona, Candida, Aspergillus* and any combination thereof; preferably the prosthetic infection is caused by microorganisms of genera selected from *Staphylococcus, Escherichia, Klebsiella, Salmonella, Serratia* and any combination thereof; more preferably the infection is caused by microorganisms selected from *Staphylococcus aureus, Staphylococcus epidermidis, Staphylococcus pyogenes, Staphylococcus agalactiae, Escherichia coli, Klebsiella pneumoniae* and any combination thereof.

A fourth aspect of the present invention relates to the bone cement, as described earlier, for use in surgery.

A fifth aspect of the present invention relates to the use of the bone cement, as described earlier, for manufacturing a prothesis or a spacer.

A sixth aspect of the present invention relates to a kit for manufacturing the bone cement, as described earlier, comprising:

- a first component comprising polymethylmethacrylate, and/or a second component comprising methyl methacrylate; and
- microcapsules containing an antimicrobial selected from rifampicin, rifabutin, rifapentine, rifalazil and any combination thereof.

The microcapsules comprise alginate.

Acrylic polymer is understood to be polymers based on the structure of acrylic acid, such as for example polymethylmethacrylate. An acrylic monomer is a monomer based on the structure of acrylic acid, such as methyl methacrylate, butylmethacrylate, etc.

In an embodiment of the sixth aspect of the present invention, the kit comprises at least the first component and the microcapsules that contain the antimicrobial are mixed with the first component.

A seventh aspect of the present invention relates to a procedure for obtaining the bone cement, as described earlier, that comprises the following stages:

a) mixing a first component comprising polymethylmethacrylate with microcapsules containing the antimicrobial selected from rifampicin, rifabutin, rifapentine, rifalazil, and any combination thereof;
b) adding the mixture of stage (a) to a second component that comprises methyl methacrylate.

## DESCRIPTION OF THE DRAWINGS

Figure 1 shows micrographs obtained by scanning electron microscopy (SEM) of the alginate microcapsules (A) and PHBV (B) loaded with rifampicin.

Figure 2 shows rifampicin eluded in PBS for cements with alginate (Alg) microcapsules, with PHBV (PHBV) microcapsules and cements with unencapsulated rifampicin (Rif).

Figure 3 shows Shore D Hardness (D) with the setting time for cement without rifampicin, control (C), cement with unencapsulated rifampicin (RIF), cement with rifampicin encapsulated in PHBV (PHBV) microcapsules and cement with rifampicin encapsulated in alginate (Alg) microcapsules.

Figure 4 shows Petri dishes wherein the inhibition halos of *Staphylococcus aureus* produced by the samples of cement with alginate (Alg), PHBV (PHBV) and unencapsulated rifampicin (C) microcapsules can be observed.

## EXAMPLES

Following is an illustration of the invention by means of assays conducted by the inventors which demonstrate the effect of the microencapsulation of the antibiotic on the mechanical characteristics of and the microbiological growth

inhibition capacity of the cement of the invention.

In examples 3-8 the examples comprising alginate microcapsules are according to the claimed invention. The examples including PHBV microcapsules are to be considered as reference examples.

**Example 1 - Microencapsulation with sodium alginate**

The rifampicin used in this experimental study was Rifaldin® 600 mg injectable IV (Sanofi, Barcelona, Spain). The packet includes a vial containing freeze-dried powder and an ampoule containing solvent.

The microcapsules were synthesised by ionic gelification. This method is based on the cross-linking capacity of the polyelectrolytes to form hydrogels in the presence of counter-ions. Alginate is the polyanionic polysaccharide most commonly used in ionic gelification (ionotropic). This method consists of dissolving the compound/antibiotic to be encapsulated in an aqueous solution of sodium alginate. Subsequently, said mixture is added to an aqueous solution containing the counter-ion (such as a $CaCl_2$/chitosan solution). The addition can be carried out by means of different procedures such as syringe drip, various vibration systems, air spraying for achieving extrusion of the polysaccharide solution, which makes it possible to obtain smaller particle sizes. When the drop of sodium alginate comes into contact with the solution containing the counter-ions (generally $Ca^{2+}$), instant gelification of the polysaccharide occurs, giving rise to the formation of a membrane or coating. The ionic gelification method can be carried out directly or indirectly, also called internally, when the counter-ion is released by an insoluble complex present in the polysaccharide solution.

In order to prepare the rifampicin-loaded alginate microcapsules, 100 mL of a 1.5% sodium alginate solution containing 200 mg of antibiotic were initially prepared with an alginate:RIF ratio of 7.5:1. Next, this solution was added, using a micro-syringe with an inner diameter of 0.05 mm, to a cross-linking aqueous solution containing 0.6% of $CaCl_2$. Additionally, in order to minimise capsule porosity and, therefore, the loss or migration of rifampicin during their formation, 0.5% chitosan was added to the cross-linking solution.

Table 1. Initial quantities of alginate and rifampicin and production yield of the different alginate microcapsule samples.

| Example | Alginate (g) | Rifampicin (g) | Yield |
|---|---|---|---|
| 1.1 | 1.51 | 0.21 | 120% |
| 1.2 | 1.50 | 0.20 | 127% |
| 1.3 | 1.50 | 0.20 | 133% |
| 1.4 | 1.50 | 0.20 | 138% |
| 1.5 | 1.50 | 0.20 | 157% |
| Average | 1.50 | 0.20 | 135% |

The production yield of microcapsules in the alginate samples was calculated as the ratio between the quantity of product obtained (microcapsule mass) between the quantity of initial reagents (rifampicin mass + alginate mass). The yield obtained was greater than 100% due to the fact that the calcium and chitosan contained in the cross-linking solution form part of the microcapsule. It could not be quantified separately.

In order to determine the rifampicin content of the microcapsules (percentage by weight), 5 mg of each sample were dissolved in 5 ml of ethylendiaminotetraacetic acid (AEDT) 0.25 M. In order to favour the total dissolution of the rifampicin, the microcapsules with the solvent were centrifuged at 4,000 rpm for 5 minutes. The supernatant was analysed using UV-visible spectrophotometry at a wavelength $\lambda$ of 334 nm, subsequent to building the corresponding calibration curve. Each of the samples was analysed in triplicate and the average rifampicin concentration values in the supernatant are shown in Table 2.

Therefore, the % of rifampicin (contained in the active ingredient or encapsulation capacity) makes reference to the quantity of rifampicin in the microcapsules, calculated as follows:

$$\% \text{ Rifampicin} = \text{Rifampicin mass} / \text{microcapsule mass} \times 100$$

Table 2. Average values of the concentrations (μg/ml), standard deviation and percentage of rifampicin in each of the four samples of alginate microcapsules.

| Example | C (μg/ml) | σ | % Rifampicin in microcapsules |
|---|---|---|---|
| 1.1 | 69.40 | 6.09 | 6.94 |
| 1.2 | 60.32 | 5.15 | 6.03 |
| 1.3 | 58.40 | 4.32 | 5.84 |
| 1.4 | 35.78 | 2.44 | 3.58 |
| 1.5 | 47.58 | 2.46 | 4.76 |
| Average | 54.29 | 4.09 | 5.43 |

The encapsulation yield is the quotient between total encapsulated rifampicin and initial rifampicin, multiplied by 100.

$$\eta_{\text{Rif Enc}} = \text{Rifampicin mass in microcapsules / Initial rifampicin mass x 100}$$

Table 3. Rifampicin encapsulation yield in the alginate microcapsules.

| Example | Initial rif (g) | Encapsulated rif (g) | Encapsulation yield |
|---|---|---|---|
| 1.1 | 0.21 | 0.14 | 69.2% |
| 1.2 | 0.20 | 0.13 | 65.4% |
| 1.3 | 0.20 | 0.12 | 60.1% |
| 1.4 | 0.20 | 0.084 | 42.1% |
| 1.5 | 0.20 | 0.13 | 63.4% |
| Average | 0.20 | 0.12 | 60.0% |

**Reference Example 2 - Microencapsulation with PHBV**

They were synthesised by the polymer precipitation by solvent evaporation method. Microencapsulation by precipitation by solvent evaporation consists of the emulsification of a solution that contains the coating polymer and the substance intended to be encapsulated in an additional medium wherein both cannot be soluble. The technique is relatively simple and has been used to prepare a wide variety of microcapsules on different substances with multiple coating materials.

In order to obtain microcapsules by solvent evaporation, the selected quantity of coating material (PHBV) was dissolved in 30 mL of chloroform with the help of magnetic agitation. Next, the antibiotic was added and agitated until achieving its complete dissolution. A polymer:RIF ratio of 7.5:1 was used. Next, this solution was dispersed in 200 mL of an aqueous solution containing 2% surfactant. In this case, the surfactant selected was polyvinylalchohol (PVA). The dispersal was carried out by means of a mechanical agitator at 2,000 rpm. Upon adding the solution containing the coating polymer and the antibiotic, it is left in agitation and controlling the temperature by means of a water bath at 30°C for 2 hours to achieve the complete evaporation of the solvent, thereby allowing the coating polymer to precipitate on the antibiotic and achieve the formation of microcapsules. Lastly, the microcapsules obtained were filtered, washed and left to air dry.

After preparing eight different samples of microcapsules containing rifampicin, the microcapsule production yields detailed in the table below were obtained:

Table 4. Initial PHBV and rifampicin candidates and production yield of PHBV microcapsules.

| Example | PHBV (g) | Rifampicin (g) | Yield |
|---|---|---|---|
| 2.1 | 0.96 | 0.12 | 73.25% |
| 2.2 | 0.91 | 0.11 | 57.12% |

(continued)

| Example | PHBV (g) | Rifampicin (g) | Yield |
|---|---|---|---|
| 2.3 | 0.93 | 0.12 | 71.64% |
| 2.4 | 0.90 | 0.11 | 66.00% |
| 2.5 | 0.94 | 0.12 | 73.29% |
| 2.6 | 0.92 | 0.13 | 77.90% |
| 2.7 | 0.89 | 0.12 | 75.70% |
| 2.8 | 0.90 | 0.10 | 70.39% |
| 2.9 | 0.90 | 0.10 | 70.42% |
| Average | 0.92 | 0.11 | 70.63% |

In order to determine the rifampicin content of the microcapsules, 5 mg of each sample were dissolved in 5 ml of a dichloromethane (10%) and methanol (90%) solution. In order to favour the total dissolution of the rifampicin, the microcapsules with the solvent were centrifuged at 4,000 rpm for 5 minutes. The surfactant was analysed by means of UV-visible spectrophotometry subsequent to building the corresponding calibration curve. Each of the samples were analysed in triplicate. The table below shows the average concentration values of rifampicin in the surfactant.

Table 5. Average rifampicin concentration values, standard deviation ($\sigma$) and percentage of encapsulated rifampicin in each of the eight PHBV microcapsule samples.

| Eca | $C_m$($\mu$g/ml) | $\sigma$ | % Rifampicin in microcapsules |
|---|---|---|---|
| 2.1 | 20.40 | 1.89 | 2.04 |
| 2.2 | 18.20 | 1.71 | 1.82 |
| 2.3 | 26.10 | 0.61 | 2.61 |
| 2.4 | 15.17 | 0.32 | 1.52 |
| 2.5 | 19.30 | 0.79 | 1.93 |
| 2.6 | 15.80 | 0.96 | 1.58 |
| 2.7 | 15.63 | 2.95 | 1.56 |
| 2.8 | 15.97 | 1.01 | 1.59 |
| 2.9 | 21.17 | 0.78 | 2.12 |
| Average | 18.64 | 1.22 | 1.86 |

Table 6. Rifampicin encapsulation yield in PHBV microcapsules.

| Example | Initial rif (g) | Encapsulated rif (g) | Encapsulation yield |
|---|---|---|---|
| 2.1 | 0.12 | 0.016 | 13.53% |
| 2.2 | 0.11 | 0.009 | 8.55% |
| 2.3 | 0.12 | 0.019 | 16.05% |
| 2.4 | 0.11 | 0.010 | 9.43% |
| 2.5 | 0.12 | 0.015 | 12.69% |
| 2.6 | 0.13 | 0.013 | 9.98% |
| 2.7 | 0.12 | 0.012 | 10.40% |
| 2.8 | 0.10 | 0.011 | 9.79% |
| 2.9 | 0.10 | 0.015 | 14.63% |

(continued)

| Example | Initial rif (g) | Encapsulated rif (g) | Encapsulation yield |
|---|---|---|---|
| Average | 0.11 | 0.013 | 11.67% |

Of the two first polymers shown in the examples, it can be said that the microcapsules that contain the highest percentage of rifampicin are the alginate microcapsules, 5.4% on average, while the PHBV microcapsules only contain 1.75% on average.

**Example 3** - **Elution of the microcapsules**

Elution was carried out in phosphate buffered saline (PBS) in order to check whether rifampicin is capable of eluting from the microcapsules towards a medium similar to the physiological medium

5 mg of each microcapsule sample were immersed in 5 ml of PBS and the suspensions were incubated at 37°C in a heater, extracting aliquots of 1 ml after 6 h, 24 h, 48 h and 1 week. The millilitre extracted for each of these times was replaced with PBS and subsequently taken into account in the calculations. Prior to taking the aliquot, the mixture was homogenised and left to stand until the microcapsules sedimented, such that as to take the aliquota without removing microcapsules.

The concentration of rifampicin eluted by the PHBV microcapsules for each one of the times studied is shown in Table 7 and for the alginate microcapsules in Table 8. Three analyses of each of the three different samples 1, 3 and 3 were performed for the PHBV and 4, 5 and 6 for the ALG, which correspond to different production lots. The PHBV microcapsule samples used in the study contained 2.04% (example 2.1), 1.82% (example 2.2) and 2.61% (example 2.3), of rifampicin. The alginate microcapsule samples contained 6.94% (example 1.1), 6.03% (example 1.2) and 5.84% (example 1.3) of rifampicin.

Table 7. Average concentration ($C_m$) of eluted rifampicin with time and standard deviation ($\sigma$) for PHBV microcapsules.

| | 6 h | | 24 h | | 48 h | | 1week | |
|---|---|---|---|---|---|---|---|---|
| | $C_m$ ($\mu$g/ml) | $\sigma$ | $C_m$ ($\mu$g/ml) | $\sigma$ | $C_m$ ($\mu$g/ml) | $\sigma$ | $C_m$ ($\mu$g/ml) | $\sigma$ |
| 2.1 | 10,53 | 0,81 | 17,67 | 2,65 | 16,40 | 1,60 | 16,17 | 2,25 |
| 2.2 | 10,23 | 1,36 | 14,10 | 0,2 | 18,33 | 2,25 | 17,23 | 1,02 |
| 2.3 | 8,23 | 0,95 | 12,73 | 1,68 | 13,63 | 3,25 | 19,10 | 1,21 |
| Average | 9,66 | 1,25 | 14,83 | 2,55 | 16,12 | 2,60 | 17,50 | 1,48 |

Table 8. Average concentrations ($C_m$) and standard deviation ($\sigma$) of the eluted rifampicin of the alginate microcapsules.

| | 6 h | | 24 h | | 48 h | | 1 week | |
|---|---|---|---|---|---|---|---|---|
| | $C_m$ ($\mu$g/ml) | $\sigma$ | $C_m$ ($\mu$g/ml) | $\sigma$ | $C_m$ ($\mu$g/ml) | $\sigma$ | $C_m$ ($\mu$g/ml) | $\sigma$ |
| 1.1 | 35.10 | 6.23 | 55.70 | 6.82 | 71.07 | 11.32 | 78.17 | 7.34 |
| 1.2 | 27.50 | 1.23 | 47.20 | 2.90 | 56.63 | 2.53 | 51.03 | 3.51 |
| 1.3 | 35.97 | 5.96 | 54.80 | 2.10 | 72.70 | 3.83 | 70.87 | 3.75 |
| Average | 32.85 | 4.66 | 52.27 | 4.67 | 66.69 | 9.03 | 66.96 | 14.04 |

As it can be observed, both the alginate and PHBV microcapsules showed good rifampicin elutions, although the alginate microcapsules showed greater elutions at all measurement times, both in total eluted quantity and in percentage with respect to the rifampicin they contained, the differences between the average rifampicin concentration values detected being statistically significant. The alginate microcapsules showed greater elutions than the rest of the microcapsules at all measurement times, the differences between the average rifampicin concentration values detected being statistically significant ($p=0.0001$).

In bone cement, the antibiotic elution should be maximum in the first hours, reaching the minimum inhibitory concentration (MIC).

Table 9. Percentage of rifampicin eluted from the microcapsules in the first hours with respect to the total rifampicin they contained and average elution values.

| | PHBV | | | ALG | | |
|---|---|---|---|---|---|---|
| Elution time | 2.1 | 2.2 | 2.3 | 1.1 | 1.2 | 1.3 |
| 6 h | 51.63% | 56.23% | 31.55% | 48.78% | 56.31 % | 61.02% |
| Average (6 h) | 46.47% | | | 55.37% | | |
| σ(6 h) | 13.13% | | | 6.17% | | |
| 24 h | 86,60% | 77.47% | 48.79% | 77.84% | 96.60% | 93.79% |
| Average (24 h) | 70.95% | | | 89.41 % | | |
| σ(6 h) | 19.73% | | | 10.12% | | |

**Example 4** - **Preparation of the bone cement**

In order to carry out this study, bone cement cores were manufactured, to which microcapsules of different rifampicin-loaded polymers were added. The composition of the bone cement used is based on the DePuy CMW1 (Ormsby, R. et al., J Mech Behav Biomed Mater. Vol. 3, No. 2, 02.2010, p. 136-145). They were sterilised by gamma irradiation.

Its composition is detailed in the table below:

Table 10. Composition of the bone cement used in these studies. For the cement elution assays and mechanical assays, test cores were prepared with the following bone cement samples:

| Bone cement | Components | Quantity |
|---|---|---|
| Powder phase | | **39.9 g** |
| Colacryl® B866 | Polymethylmethacrylate (PMMA) | 36.36 g |
| In itiator | Benzoyl peroxide (BPO) (1-5% included in the PMMA) | |
| Radiopaque agent | Barium sulphate ($BaSO_4$) | 3.54 g |
| Liquid phase | | **19.4 ml** |
| Monomer | Methylmethacrylate (MMA) | 19.4 ml |
| Activator | N,N-Dimethyl-p-toluidine | 160 μl |

Table 11. Prepared cement samples. Three different lots of each sample were prepared for each type of assay.

| | |
|---|---|
| Control | Control bone cement. Composition of Table 10 |
| Rif | Control bone cement + unencapsulated rifampicin (Rif) |
| PHBV | Control bone cement + poly-hydroxybutyrate-hydroxyvaleerate microcapsules with rifampicin |
| ALG | Control bone cement + alginate microcapsules with rifampicin |

The average temperature at the time of performing the mixture was 23±2°C and relative humidity was 55±10%. Rifampicin or the rifampicin-loaded microcapsules were manually mixed with the component in powder form of the cement in accordance with the method of Frommelt, et al., (Properties of bone cement: antibiotic-loaded cement. In: Breusch S, Malchau H, editors. The well-cemented total hip arthroplasty. Berlin Heidelberg: Springer-Verlag; p. 86, 2005) in a tray and, once homogenised, the liquid component was added until a complete mixture was achieved.

**Example 5** - **Elution of the rifampicin-loaded bone cement**

In order to elute the bone cement loaded with rifampicin microcapsules manufactured with PHBV and alginate polymer coatings and with unencapsulated rifampicin, cylindrical cement cores 6 mm in diameter and 12 mm in height were manufactured in predesigned PTFE (polytetrafluoroethylene) moulds.

The bone cement samples manufactured with microcapsules contained 5% w/w of microcapsules. The samples manufactured with unencapsulated rifampicin contained 1.25% w/w of rifampicin. The ratio was:

- 40 g of cement with 0.5 g of unencapsulated rifampicin;
- 40 g of cement with 2 g of PHBV or alginate microcapsules.

The cylindrical cement cores were left to set for at least 24 hours, after which they were immersed in a sealed test tube (tube 1) with 5 ml of PBS and incubated at 37°C in the absence of light. After 6 hours had elapsed, the cylinder was removed from the tube and introduced in another tube containing 5 ml of new PBS in order to allow it to continue eluting the antibiotic.

The dissolution of tube 1 was analysed by means of UV-visible spectrophotometry (Agilent Cary 4000) to determine the quantity of rifampicin eluted within that timeframe from the cement, selecting a wavelength of 334 nm, which the most stable absorbance peak of rifampicin, using previously calculated calibration line. The same process was repeated for the elution times of 24 h, 48 h and 1 week.

Table 12. Average concentrations and standard deviation ($\sigma$) of eluted rifampicin eluted by the rifampicin-loaded cement cores, with PHB microcapsules and alginate microcapsules, at different measurement time.

| | 6 h | | 24 h | | 48 h | | 1 week | |
|---|---|---|---|---|---|---|---|---|
| | $C_m$ (μg/ml) | $\sigma$ | $C_m$ (μg/ml) | $\sigma$ | $C_m$ (μg/ml) | $\sigma$ | $C_m$ (μg/ml) | $\sigma$ |
| Rif | 0 | 0.00 | 0 | 0.00 | 0 | 0.00 | 0.6 | 0.082 |
| PHBV | 0.01 | 0.032 | 0.01 | 0.00 | 0.01 | 0.00 | 0.27 | 0.16 |
| ALG | 1.96 | 1.52 | 2.45 | 1.45 | 1.25 | 0.98 | 3.43 | 2.53 |

Unencapsulated rifampicin-loaded cements do not elute antibiotic concentrations measurable in the first hours (0.0% in 48 h) and after one week the quantity eluted is 0.05% of their total rifampicin content (0.6 μg/ml). Cements containing PHBV microcapsules elute a small quantity of antibiotics in the first hours, 0.01% in 6 h (0.01 μg/ml), but more than in the case of unencapsulated PHBV, achieving an elution of 0.3% of the total in one week (0.27 μg/ml). Cements containing alginate microcapsules elute 0.68% of the antibiotic they contain in the first 6 hours (1.96 μg/ml), achieving an elution of 3.38% after one week (3.43 μg/ml). In the case of cements containing PHBV or alginate microcapsules, from the first hours the quantity of antibiotic eluted exceeded the MIC of *Staphylococcus aureus* (0.008 μg/ml). The cement samples with alginate microcapsules showed greater rifampicin elution than the cement samples containing PHBV microcapsules and the cement samples containing unencapsulated rifampicin, at all measurement times, wherein the differences were statistically different ($p<0.01$).

The total eluted or accumulated rifampicin concentrations for these three cases are represented in figure 2.

**Example 6** - **Setting time assays**

The essential claim of this invention consists of manufacturing rifampicin-loaded bone cement, which is capable of setting within a reasonable timeframe to be used in the operating room, i.e. approximately 15 minutes, which is the setting time of commercial cements.

In order to verify that the cement of the invention allows this quick setting thanks to the microencapsulation of the rifampicin and which, in the event of not being encapsulated, setting is much slower, various cores were manufactured with cement without antibiotics (control, C), with 1.25% of unencapsulated rifampicin (RIF), cement with 5% of PHBV microcapsules with rifampicin (PHBV) and cement with 5% of alginate microcapsules with rifampicin (ALG).

A predesigned silicone mould was used to obtain rectangular samples 8.0±0.1 mm long, 10.0±0.1 wide and 4.0±0.1 mm thick, according to the ISO 5833:2002 international standard. Upon being removed from the mould, the samples were manually prepared using a file to remove any possible macroscopic irregularities and achieve their homogeneity. Cores with macroscopic bubbles were discarded.

The cement setting status was verified by means of a Shore D hardness test following the UNE-ISO 7619-1:2011 international standard every 15 min during the first hour, after 2 h, 3 h, 4 h and 24 h subsequent to the manufacture of

the cement. Three measurements were taken for each of the times and groups. The Shore hardness tester was used (BAREISS, Neutek, Eibar, Spain).

The hardness of the cement containing alginate microcapsules was similar in all the measurement times to that of the control cement, achieving maximum hardness from the first measurement interval (15 min). The cement containing PHBV microcapsules showed less hardness in the first two hours but was subsequently similar to that of the cement containing alginate microcapsules and to that of the control cement. However, the cement containing unencapsulated rifampicin is barely consistent 15 minutes after being prepared and is far from the hardness of the other three up to a setting time of 45 minutes. For any of the times in which hardness was measured, this cement always gave a lower value, as observed in figure 3.

Bending tests were conducted after the first 45 min of setting for the four previous types of cement (control, RIF, PHBV and Alg), preparing five samples of each type of cement in order to determine the theoretical resistance of the cement shortly after the operation.

To this end, test cores were prepared in a similar manner to those used in the hardness tests. They were all subject to a four-point bending test using an IBTH 500 machine (Ibertest, Madrid, Spain) with a 5 kN load cell at a testing speed of 5 mm/min using an Epsilon 3540 deflectometer. Force with respect to movement was recorded during each assay in order to subsequently determine tensile strength as well as the bending modulus of each of the test cores pursuant to the ISO 5833:2002 standard.

The resistance and bending modulus results are shown in tables 13 and 14. When unencapsulated rifampicin is added to the cement, its resistance and flexion modulus decreases by more than 50%. However, when the rifampicin is encapsulated, even if the resistance worsens, it is only by approximately 20%, the bending modulus in the case of alginate being very similar to that of the control cement with a small decrease of 7.5%.

Table 13. Average bending resistance values (MPa), standard deviation (σ) and reduction in the resistance of the cements studied with respect to the control cement after setting for 45 minutes.

| | Bending resistance (MPa) | σ | Variation with respect to the control |
|---|---|---|---|
| Control | 50.93 | 1.76 | |
| RIF | 22.87 | 6.84 | -55.09% |
| PHBV | 39.36 | 4.36 | -22.71 % |
| Alginate | 40.43 | 4.72 | -20.61 % |

It can therefore be observed that the encapsulation of rifampicin enables its use as an antibiotic in the local treatment of periprosthetic infections, in spacers, since the cement reaches sufficient consistency and resistance within a reasonable timeframe after the operation.

Table 14. Average values of the bending moduli (MPa) of the cement samples studied, standard deviation (σ) and reduction with respect to the control, after setting for 45 minutes.

| | Bending modulus (MPa) | σ | Variation with respect to the control |
|---|---|---|---|
| Control | 2333 | 41 | |
| RIF | 954 | 279 | -59.11% |
| PHBV | 1844 | 216 | -20.96% |
| Alginate | 2158 | 195 | -7.5% |

**Example 7 - Resistance of the bone cement after complete setting**

In order to verify that the bone cement of the invention has sufficient mechanical resistance to be used as a prosthesis fixer and as a spacer, mechanical bending and compression tests were conducted one week after setting thereof, during which time it reached its maximum resistance.

The bending test was that described previously following the ISO 5833:2002 standard. The results can be observed in the following tables:

Table 15. Average bending resistance values of the cements studied (MPa), standard deviation (σ) and reduction in resistance with respect to the control cement, after one week of setting.

| | Bending resistance (MPa) | σ | Variation with respect to the control |
|---|---|---|---|
| Control | 54.66 | 2.82 | |
| RIF | 46.04 | 3.79 | -16% |
| PHBV | 49.46 | 5.95 | -9.5% |
| Alginate | 44.72 | 3.99 | -18.2% |

The greatest bending resistance was observed in the control cement cores (without rifampicin). The bending resistance of the cement containing PHBV microcapsules was reduced by 9.5% with respect to the control cement. In the case of cement containing unencapsulated rifampicin, bending resistance decreased by 16%. The least bending resistance was observed in the cement containing alginate microcapsules (decrease of 18.2%).

The average values and standard deviation of the bending modulus were calculated, obtained from the cement cores studied, observing that the microencapsulation of rifampicin gives rise to a smaller variation in the bending modulus of the bone cement, in comparison with the unencapsulated rifampicin. In fact, microencapsulation with PHBV and alginate did not alter the elasticity modulus with respect to the control cement.

Table 16. Average values of the bending modulus of the cements studied (MPa), standard deviation (σ) and variation of the modulus with respect to the control cement, after one week of setting.

| | Bending modulus (MPa) | σ | Variation with respect to the control |
|---|---|---|---|
| Control | 2898 | 496 | |
| RIF | 2371 | 207 | -18.18% |
| PHBV | 3026 | 433 | 4.4% |
| Alginate | 2937 | 245 | 1.3% |

In order to conduct the compression tests, the ISO 5833:2002 international standard was followed. Cylindrical cores 12.0±0.1 mm high and 6.0±0.1 mm in diameter were manufactured. One week after the manufacture thereof they were tested at a compression speed of 20 mm/min and with a 20 kN cell.

Each core was assayed until failure using a Universal Testing Machine ELIB 20W (Ibertest, Madrid, Spain). Tensile strength under compression was calculated as specified in the ISO 5833:2002 Bone Cement standard.

Table 17. Average compression resistance values for the cements studied (MPa), standard deviation (σ) and reduction in resistance with respect to the control cement, after one week of setting.

| | Compression resistance (MPa) | σ | Variation with respect to the control |
|---|---|---|---|
| Control | 106.2 | 2.97 | |
| RIF | 74.04 | 3.57 | -30% |
| PHBV | 91.26 | 5.13 | -13.9% |
| Alginate | 91.35 | 6.29 | -13.8% |

Cements containing PHBV microcapsules and alginate showed less resistance to compression than the control cement (cement without addition of antibiotics) with a reduction of 14%. Cement containing unencapsulated rifampicin showed a reduction in compression resistance of 30% with respect to the control cement.

A reduction of 10% to 20% in the bending and compression resistance of the antibiotic-loaded cement is feasible. It has been demonstrated that the addition of antibiotic to bone cement generally entails a reduction in its resistance, said reduction depending on the quantity and type of antibiotic added (Paz E, Sanz-Ruiz P, The Journal Of Arthoplasty, 30:1423-1429, 2015; Hendriks et al. Biomaterials;2 5(3):545, 2004). For example, Baleani et al. concluded that the addition of 0.5 g of vancomycin and 0.5 g of meropenem to bone cement causes a reduction of more than 15% in bending resistance, while the addition of 1 g of vancomycin reduces this value by more than 20% (Baleani M, et al. The Journal of arthroplasty, 23(8):1232-8, (2008)).

Therefore, it can be said that the microencapsulation of rifampicin with PHBV and alginate, and probably with other polymers, produces cements with sufficient bending and compression resistance to be used for fixing prostheses and as spacers.

**Example 8 - Microbiological assays**

Bone cement cores 2 mm thick and 12 mm in diameter containing unencapsulated rifampicin, PHBV microcapsules and alginate microcapsules were manufactured in predesigned silicone moulds. The samples contained 5% w/w of microcapsules (PHBV and alginate) or 1.25% w/w of rifampicin.

Eight Petri dishes with blood agar which were previously inoculated with *Staphylococcu aureus,* subspecies *aureus,* ATCC® 29213 ™ (Manassas, USA) were used to determine the antimicrobial activity of the cement samples.

A sample of the microorganism was taken and diluted in sterilised water in a test tube until reaching a concentration compliant with the 0.5 McFarland Standard using a turbidimeter (Microscan®, Baxter, West Sacramento, CA, USA). Next, the Petri dishes were uniformly sown using an inoculating loop in three directions with 60° turns. Cement samples containing unencapsulated rifampicin were placed on two dishes, cement samples containing alginate microcapsules (corresponding to three different lots of microcapsules) on three dishes and cement samples containing PHBV microcapsules on another three dishes (corresponding to three different lots of microcapsules).

Each cement sample was placed in the centre of a Petri dish using sterile pincers. It was subjected to incubation in a heater at 37°C for 24 h. The inhibition halo was determined in mm 24 h after incubation. This system is based on the disk diffusion method (Baeur-Kirby), which proves the inhibition or resistance of the microorganisms by subjecting them to the antibiotic in question. The presence of the inhibition halo indicates that the microencapsulated rifampicin was capable of eluting from the microcapsules and bone cement, diffusing in the blood agar medium and inhibiting the growth of *S. aureus.*

Table 18. Average inhibition halo values (mm) and standard deviation for each group. N: number of measured samples.

| | N | Average (mm) | $\sigma$ |
|---|---|---|---|
| RIF | 6 | 9.33 | 4.41 |
| Alginate | 9 | 20.78 | 3.23 |
| PHBV | 9 | 4.89 | 2.93 |

The inhibition halos observed in the cement sample containing alginate microcapsules were larger than in the cement samples containing PHBV microcapsules and than those of the cement samples containing unencapsulated rifampicin, despite the fact that these last samples contain a larger amount of antibiotic (1.25% compared to 0.3% of the cement with alginate). Statistically significant differences were observed on comparing the diameters of the inhibition halos of the cement samples containing alginate with those of the cement samples containing rifampicin or PHBV (p=0.0001) (fig. 4).

**Claims**

1. Bone cement comprising microcapsules containing rifampicin, rifabutin, rifapentine, rifalazil and any combination thereof wherein the bone cement is an acrylic-based bone cement with a polymethylmethacrylate base and wherein the microcapsules are formed by alginate and are obtained by ionic gelification.

2. The bone cement, according to claims1 wherein the microcapsules have a diameter between 2 $\mu$m and 2 mm.

3. The bone cement, according to any of the preceding claims, wherein the concentration of the microcapsules is between 0.1% and 20% by weight with respect to the total weight of the bone cement.

4. The bone cement, according to any of the preceding claims, wherein the concentration of the antimicrobial is between 0.01% and 10% by weight with respect to the total weight of the bone cement.

5. The bone cement, according to any of the preceding claims, for use in therapy.

6. The bone cement, according to any of claims 1 to 4, for use in the treatment and/or prevention of prosthetic infections.

7. The bone cement, for use according to claim 5 or 6, wherein the prosthetic infection is caused by microorganisms of genera selected from *Staphylococcus, Propionibacterium, Enterococcus, Streptococcus, Alishewanella,Alterococcus, Aquamonas, Aranicola, Arsenophonus, Azotivirga, Blochmannia, Brenneria, Buchnera, Budvicia, Buttiauxela, Cedecea, Citrobacter, Dickeya, Edwardsiella, Enterobacter, Erwinia, Escherichia, Ewingella, Grimontella, Hafnia, Klebsiella, Kluyvera, Leclercia, Leminorella, Moellerella, Morganella, Obesumbacterium, Pantoea, Paracolobactrum, Pectobacterium, Phlomobacter, Photorhabdus, Plesiomonas, Pragia, Proteus, Providencia, Rahnella, Raoultella, Salmonella, Samsonia, Serratia, Shigella, Sodalis, Tatumella, Trabulsiella, Wigglesworthia, Xenorhabdus, Yersinia, Yokenella, Corynebacterium, Pseudomona, Candida, Aspergillus* and any combination thereof.

8. The bone cement, according to any of claims 1 to 4, for use in surgery.

9. Use of the bone cement, according to any of claims 1 to 4, to manufacture a prothesis or a spacer.

10. A kit for manufacturing the bone cement, according to claims 1 to 4, which comprises:

- a first component comprising polymethylmethacrylate, and/or a second component comprising methylmethacrylate.; and
- alginate microcapsules containing rifampicin, rifabutin, rifapentine, rifalazil, and any combination thereof obtained by ionic gelification

11. A method for obtaining the bone cement, according to claims 1 to 4, comprising the following stages:

a) mixing a first component comprising polymethylmethacrylate with the alginate microcapsules containing the antimicrobial selected from rifampicin, rifabutin, rifapentine, rifalazil obtained by. ionic gelification, and any combination thereof; and
b) adding the mixture of stage (a) to a second component comprising methylmethacrylate.

**Patentansprüche**

1. Knochenzement, umfassend Mikrokapseln, die Rifampicin, Rifabutin, Rifapentin, Rifalazil und eine beliebige Kombination davon enthalten, wobei der Knochenzement ein Knochenzement auf Acrylbasis mit einer Polymethylmethacrylatbasis ist und wobei die Mikrokapseln durch Alginat gebildet und durch ionische Gelierung erhalten werden.

2. Knochenzement nach Anspruch 1, wobei die Mikrokapseln einen Durchmesser zwischen 2 μm und 2 mm aufweisen.

3. Knochenzement nach einem der vorhergehenden Ansprüche, wobei die Konzentration der Mikrokapseln zwischen 0,1 Gew.-% und 20 Gew.-%, bezogen auf das Gesamtgewicht des Knochenzements, beträgt.

4. Knochenzement nach einem der vorhergehenden Ansprüche, wobei die Konzentration des antimikrobiellen Mittels zwischen 0,01 Gew.-% und 10 Gew.-%, bezogen auf das Gesamtgewicht des Knochenzements, beträgt.

5. Knochenzement nach einem der vorhergehenden Ansprüche zur Verwendung in der Therapie.

6. Knochenzement nach einem der Ansprüche 1 bis 4 zur Verwendung bei der Behandlung und/oder Vorbeugung von Protheseninfektionen.

7. Knochenzement zur Verwendung nach Anspruch 5 oder 6, wobei die Protheseninfektion durch Mikroorganismen der Gattungen verursacht wird, die ausgewählt sind aus *Staphylococcus, Propionibacterium, Enterococcus, Streptococcus, Alishewanella, Alterococcus, Aquamonas, Aranicola, Arsenophonus, Azotivirga, Blochmannia, Brenneria, Buchnera, Budvicia, Buttiauxela, Cedecea, Citrobacter, Dickeya, Edwardsiella, Enterobacter, Erwinia, Escherichia, Ewingella, Grimontella, Hafnia, Klebsiella, Kluyvera, Leclercia, Leminorella, Moellerella, Morganella, Obesumbacterium, Pantoea, Paracolobactrum, Pectobacterium, Phlomobacter, Photorhabdus, Plesiomonas, Pragia, Proteus, Providencia, Rahnella, Raoultella, Salmonella, Samsonia, Serratia, Shigella, Sodalis, Tatumella, Trabulsiella, Wigglesworthia, Xenorhabdus, Yersinia, Yokenella, Corynebacterium, Pseudomona, Candida, Aspergillus* und einer beliebigen Kombination davon.

8. Knochenzement nach einem der Ansprüche 1 bis 4 zur Verwendung in der Chirurgie.

**9.** Verwendung des Knochenzements nach einem der Ansprüche 1 bis 4 zur Herstellung einer Prothese oder eines Abstandshalters.

**10.** Kit zur Herstellung des Knochenzements nach Anspruch 1 bis 4, umfassend:

- eine erste Komponente, die Polymethylmethacrylat umfasst, und/oder eine zweite Komponente, die Methylmethacrylat umfasst; und
- Alginat-Mikrokapseln, die Rifampicin, Rifabutin, Rifapentin, Rifalazil und eine beliebige Kombination davon enthalten und die durch ionische Gelierung erhalten werden.

**11.** Verfahren zur Gewinnung des Knochenzements nach Anspruch 1 bis 4, umfassend die folgenden Schritte:

a) Mischen einer ersten Komponente, die Polymethylmethacrylat umfasst, mit den Alginat-Mikrokapseln, die das antimikrobielle Mittel enthalten, das ausgewählt ist aus Rifampicin, Rifabutin, Rifapentin, Rifalazil, und die durch ionische Gelierung erhalten werden, und eine beliebige Kombination davon; und
b) Zugeben der Mischung aus Stufe (a) zu einer zweiten Komponente umfassend Methylmethacrylat.

**Revendications**

**1.** Ciment osseux comprenant des microcapsules contenant de la rifampicine, de la rifabutine, de la rifapentine, du rifalazil et une quelconque combinaison de ceux-ci dans lequel le ciment osseux est un ciment osseux à base acrylique avec une base de polyméthacrylate de méthyle et dans lequel les microcapsules sont formées par l'alginate et sont obtenues par gélification ionique.

**2.** Ciment osseux, selon la revendication 1 dans lequel les microcapsules ont un diamètre compris entre 2 $\mu$m et 2 mm.

**3.** Ciment osseux, selon l'une quelconque des revendications précédentes, dans lequel la concentration des microcapsules est comprise entre 0,1 % et 20 % en poids par rapport au poids total du ciment osseux.

**4.** Ciment osseux, selon l'une quelconque des revendications précédentes, dans lequel la concentration de l'antibactérien est comprise entre 0,01 % et 10 % en poids par rapport au poids total du ciment osseux.

**5.** Ciment osseux, selon l'une quelconque des revendications précédentes, pour une utilisation en thérapie.

**6.** Ciment osseux, selon l'une quelconque des revendications 1 à 4, pour une utilisation dans le traitement et/ou la prévention des infections prothétiques.

**7.** Ciment osseux, pour son utilisation selon la revendication 5 ou 6, dans lequel l'infection prothétique est causée par des micro-organismes de genres choisis parmi *Staphylococcus, Propionibacterium, Enterococcus, Streptococcus, Alishewanella, Alterococcus, Aquamonas, Aranicola, Arsenophonus, Azotivirga, Blochmannia, Brenneria, Buchnera, Budvicia, Buttiauxela, Cedecea, Citrobacter, Dickeya, Edwardsiella, Enterobacter, Erwinia, Escherichia, Ewingella, Grimontella, Hafnia, Klebsiella, Kluyvera, Leclercia, Leminorella, Moellerella, Morganella, Obesumbacterium, Pantoea, Paracolobactrum, Pectobacterium, Phlomobacter, Photorhabdus, Plesiomonas, Pragia, Proteus, Providencia, Rahnella, Raoultella, Salmonella, Samsonia, Serratia, Shigella, Sodalis, Tatumella, Trabulsiella, Wigglesworthia, Xenorhabdus, Yersinia, Yokenella, Corynebacterium, Pseudomona, Candida, Aspergillus* et une quelconque combinaison de ceux-ci.

**8.** Ciment osseux, selon l'une quelconque des revendications 1 à 4, pour une utilisation en chirurgie.

**9.** Utilisation du ciment osseux, selon l'une quelconque des revendications 1 à 4, pour fabriquer une prothèse ou un espaceur.

**10.** Kit pour la fabrication du ciment osseux, selon les revendications 1 à 4, qui comprend :

- un premier composant comprenant du polyméthacrylate de méthyle, et/ou un deuxième composant comprenant du méthacrylate de méthyle ; et
- des microcapsules d'alginate contenant de la rifampicine, de la rifabutine, de la rifapentine, du rifalazil, et une

quelconque combinaison de ceux-ci obtenue par gélification ionique.

**11.** Procédé d'obtention du ciment osseux, selon les revendications 1 à 4, comprenant les étapes suivantes :

a) mélanger un premier composant comprenant du polyméthacrylate de méthyle avec les microcapsules d'alginate contenant l'antimicrobien choisi parmi de la rifampicine, de la rifabutine, de la rifapentine, du rifalazil obtenu par gélification ionique, et une quelconque combinaison de ceux-ci ; et
b) ajouter le mélange de l'étape (a) à un deuxième composant comprenant du méthacrylate de méthyle.

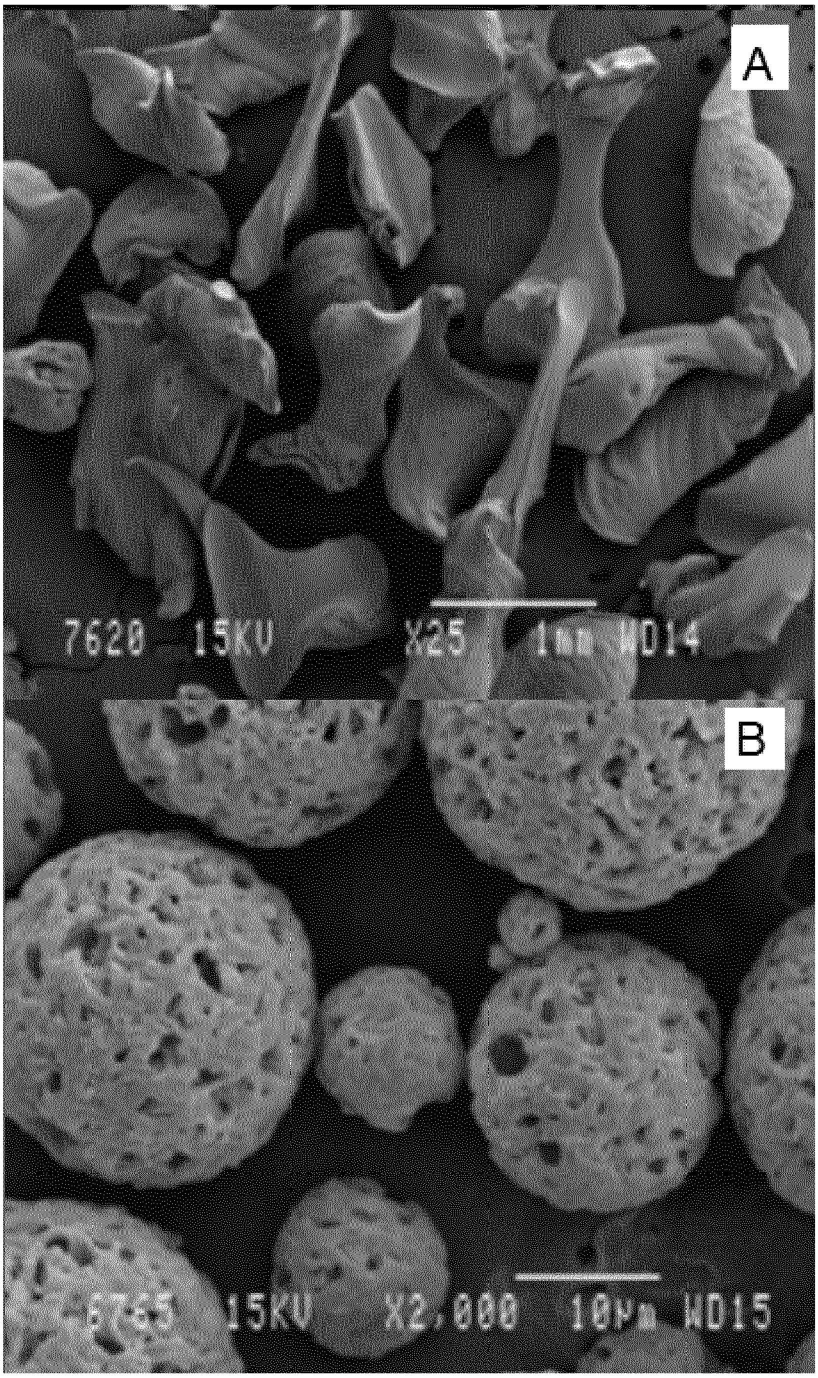


Fig. 1

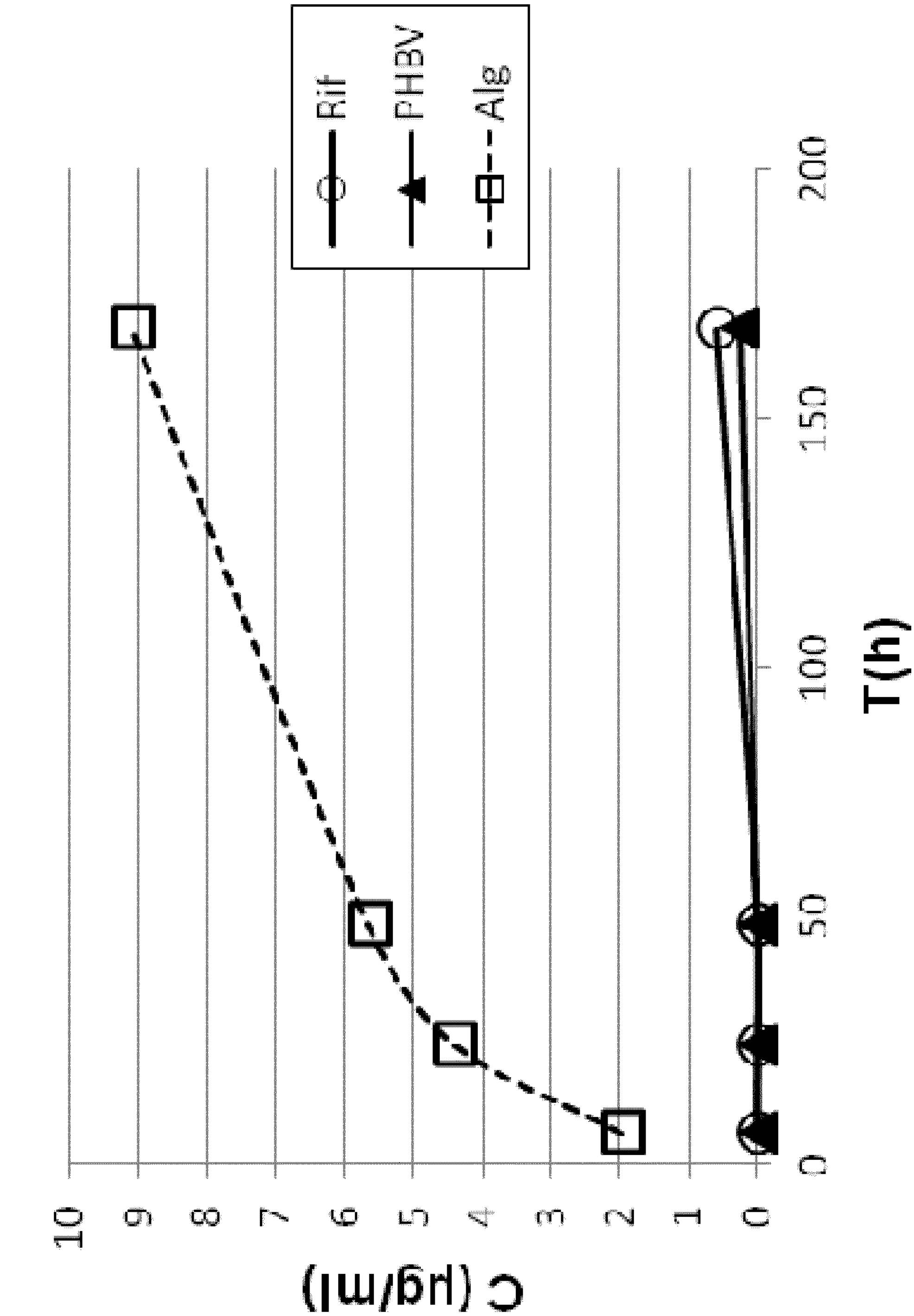


**Fig. 2**

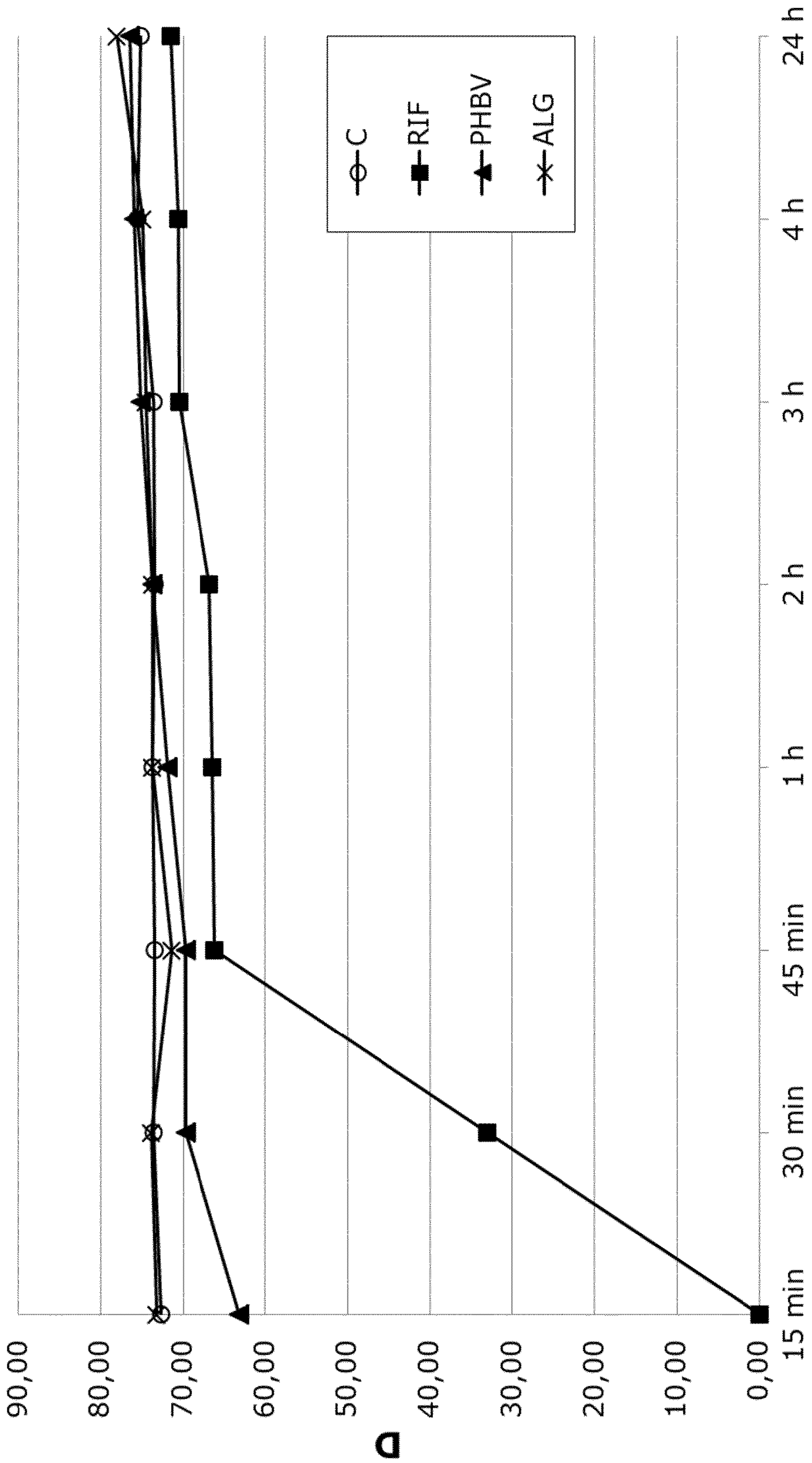
C
RIF
PHBV
ALG
D
90,00
80,00
70,00
60,00
50,00
40,00
30,00
20,00
10,00
0,00
15 min
30 min
45 min
1 h
2 h
3 h
4 h
24 h

**Fig. 3**

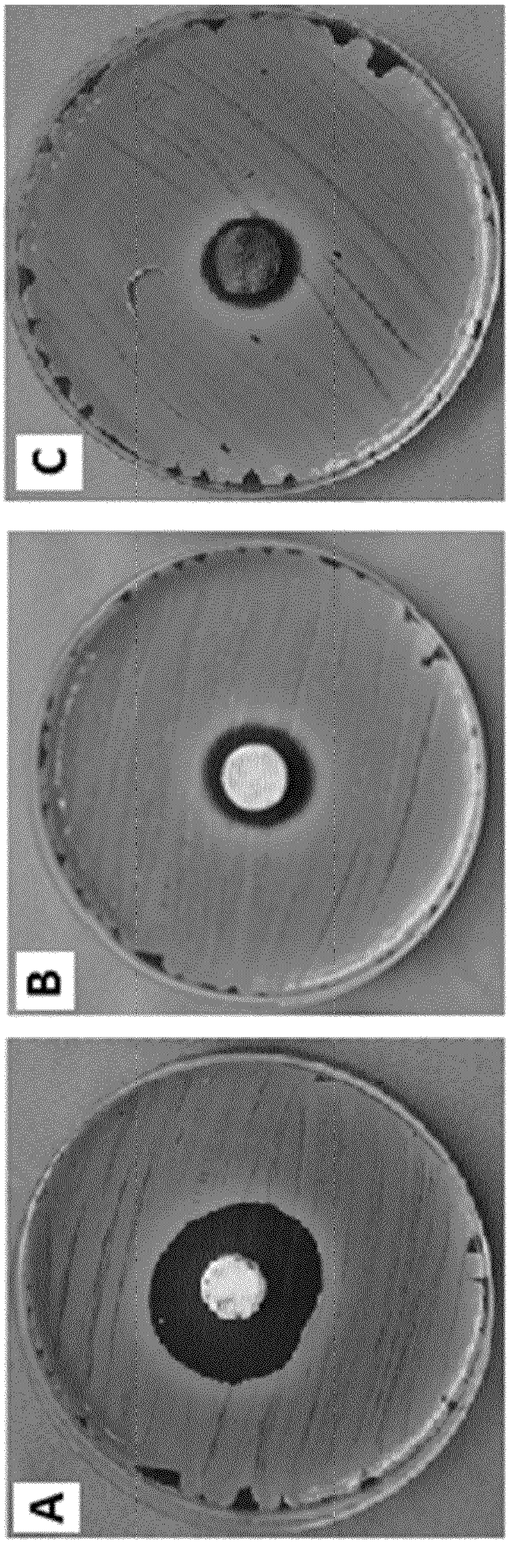


Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20150093443 A1


### Non-patent literature cited in the description


- *Acta orthopaedica,* 2008, vol. 79 (3), 335-41
- **ORMSBY, R. et al.** *J Mech Behav Biomed Mater.,* February 2010, vol. 3 (2), 136-145
- Properties of bone cement: antibiotic-loaded cement. **FROMMELT et al.** The well-cemented total hip arthroplasty. Springer-Verlag, 2005, 86
- **PAZ E ; SANZ-RUIZ P.** *The Journal Of Arthoplasty,* 2015, vol. 30, 1423-1429
- **HENDRIKS et al.** *Biomaterials,* 2004, vol. 2 5 (3), 545
- **BALEANI M et al.** *The Journal of arthroplasty,* 2008, vol. 23 (8), 1232-8